(19)

**Europäisches Patentamt**
**European Patent Office**
**Office européen des brevets**

(11)  **EP 3 627 148 A1**

(12)  **EUROPEAN PATENT APPLICATION**
published in accordance with Art. 153(4) EPC

(43) Date of publication:
25.03.2020  Bulletin 2020/13

(21) Application number: 18733782.9

(22) Date of filing: 16.05.2018

(51) Int Cl.:
*G01N 29/36* (2006.01)    *G01N 29/06* (2006.01)
*G01N 29/22* (2006.01)

(86) International application number:
PCT/KR2018/005578

(87) International publication number:
WO 2018/212573 (22.11.2018 Gazette 2018/47)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 16.05.2017  KR 20170060418

(71) Applicant: SM Instruments Co., Ltd.
Daejeon 34109 (KR)

(72) Inventors:
• KIM, Young-Ki
  Daejeon 35248 (KR)
• KIM, Young-Min
  Daejeon 34049 (KR)
• LEE, Jea-Sun
  Daejeon 34074 (KR)
• LEE, Kwang-Hyun
  Daejeon 34396 (KR)
• LIM, Jung-Hyun
  Cheonan-si
  Chungcheongnam-do 31199 (KR)
• HAN, Seong-Joo
  Uijeongbu-si
  Gyeonggi-do 11807 (KR)

(74) Representative: Ter Meer Steinmeister & Partner
Patentanwälte mbB
Nymphenburger Straße 4
80335 München (DE)

(54)  **RADIATING ULTRASONIC WAVE VISUALIZATION METHOD, AND ELECTRONIC RECORDING MEDIUM IN WHICH PROGRAM FOR PERFORMING RADIATING ULTRASONIC WAVE VISUALIZATION METHOD IS RECORDED**

(57)    A radiation ultrasonic wave visualization method in which an ultrasonic wave radiated by a sound source is visualized, comprises: heterodyne-converting ultrasonic signals in a band of at least 20 KHz or more, which are acquired by an ultrasonic Sensor array constituted by a plurality of ultrasonic Sensors and converting the ultrasonic signals into a low-frequency signal and thereafter, beamforming the converted low-frequency signals or beamforming the converted low-frequency signals based on resampling signals, thereby handling the low-frequency signals without distorting ultrasonic sound location information to reduce a data handling amount in the beamforming step.

FIG. 1A

Ultrasonic wave sensing step, in which an ultrasonic sensor array 10 constituted by a plurality N of ultrasonic sensors 11 senses ultrasonic wave signals — S110

First data acquiring step, in which a data acquiring board (DAQ board) 20 acquires ultrasonic signals $S1_a$ in an ultrasonic frequency band (20 KHz to 200 KHz) by using ultrasonic signals sensed by the ultrasonic sensor array 10 as a first sampling frequency $f_{c1}$ — S120

Low-frequency conversion signal generating step, in which a main board 30 heterodyne-converts the ultrasonic signals $S1_a$ acquired in step S20, and generates low-frequency conversion signals $S2_a$ in a sound wave band (20 Hz to 20 KHz) based on the ultrasonic signals $S1_a$ — S130

Second data acquiring step, in which the main board 30 re-samples the low-frequency conversion signals $S2_a$ generated in step S30 as a second sampling frequency $f_{c2}$, which is smaller than the first sampling frequency $f_{c1}$ to acquire a low-frequency re-sampling signal $x_a$ — S140

Sound field visualizing step, in which the main operation board 30 beam-forms the low-frequency re-sampling signals $x_a$ and a display device 70 performs the sound field visualization — S200

**(Cont. next page)**

EP 3 627 148 A1

FIG. 1B

Sound field visualizing step S200

Sound source calculating step, in which a time delay correction is applied to each of the ultrasonic signals $x_n$ using the delay distances calculated above, and sound source values $r_{nk}$ of the virtual plane points are calculated by summing up the time delay correction, after the main board 30 including an operation processing device calculates distances between the sensors 11 and virtual plane points using sensor coordinates and virtual plane coordinates — S50

Beam power level calculating step, in which the main board 30 calculates beam power levels z of the sound source values $r_{nk}$ generated in step S40 — S60

Visual display step, in which the beam power levels z calculated in step S50 are overlaid and displayed on the display device 70 together with an optical image in the direction in which the sensor array 10 is directed — S70

**Description**

## CROSS-REFERENCE TO RELATED APPLICATIONS

This application claims priority to and the benefit of Korean Patent Application No. 10-2017-0060418 filed in the Korean Intellectual Property Office on May 16, 2017, the entire contents of which are incorporated herein by reference.

## TECHNICAL FIELD

The present invention relates to a radiation ultrasonic wave visualization method and an electronic recording medium having a program for performing the radiation ultrasonic wave visualization method which is recorded therein, which are used for diagnosing a facility failure by not analyzing an echo-reflected ultrasonic wave with an ultrasonic wave transmitter and an ultrasonic wave receiver but showing a generation location of an ultrasonic wave (not an echo signal) naturally radiated from a machine or facility or a gas pipe as an image.

## BACKGROUND ART

Patent Registration No. 10-1477755 provides a high-voltage board, a low-voltage board, a distribution board, and a motor control board equipped with an ultrasonic wave-based arc and corona discharge monitoring and diagnosing system which diagnoses a discharge state of arc or corona of a housing having the high-voltage board included therein, which include a sensor unit constituted by multiple ultrasonic sensors which contact or are installed proximate to a facility provided in the housing and which detect ultrasonic waves generated by the arc or corona discharge; and a monitoring device constituting an abnormality determining unit which senses arc or corona discharge generated in the facility and controls an internal state of the housing according to the sensed arc or corona discharge information, based on an ultrasonic signal detected by the sensor unit.

## SUMMARY OF THE INVENTION

The present invention has been made in an effort to provide a radiation ultrasonic wave visualization electronic means visualizing ultrasonic waves naturally radiated by a mutual operation among components in a facility (apparatus), machines, etc. and a portable facility failure diagnosing device with a computer program, unlike a medical ultrasonic diagnosis apparatus visualizing an internal shape by a reflection wave after transmitting an ultrasonic wave by an ultrasonic apparatus in the related art.

Further, the present invention has been made in an effort to provide a radiation ultrasonic wave visualization method and an electronic recording medium having a program for performing the radiation ultrasonic wave visualization method which is recorded therein, which performs a data processing step for radiation ultrasonic wave visualization without losing ultrasonic sound source location size information in an ultrasonic area in which a data processing capacity is large and an operation processing step so as to be performed by an electronic means having appropriate performance and an operation processing capability by optimizing and minimizing a throughput.

The present invention has been made in an effort to provide a radiation ultrasonic wave visualization method and an electronic recording medium having a program for performing the radiation ultrasonic wave visualization method which is recorded therein, which can implement making as an image or output as a voice a sound of an ultrasonic area more efficient than a vibration sound which enables initial failure diagnosis in machine failure diagnosis or preliminary failure diagnosis, and monitoring the failure together with an image signal.

An exemplary embodiment of the present invention provides a radiation ultrasonic wave visualization method in which an ultrasonic wave radiated by a sound source is visualized, including: heterodyne-converting ultrasonic signals $S1_n$ in a band of at least 20 KHz or more, which are acquired by an ultrasonic sensor array 10 constituted by a plurality of (N) ultrasonic sensors 11 and converting the ultrasonic signals $S1_n$ into a low-frequency signal $S2_n$ and thereafter, beamforming the converted low-frequency signals or beamforming the converted low-frequency signals based on resampling signals $x_n$, thereby handling the low-frequency signals without distorting ultrasonic sound location information to reduce a data handling amount in the beamforming step.

Another exemplary embodiment of the present invention provides a radiation ultrasonic wave visualization method including: an ultrasonic wave sensing step (S110), in which an ultrasonic sensor array 10 constituted by a plurality N of ultrasonic sensors 11 senses ultrasonic wave signals; a first data acquiring step (S120), in which a data acquiring board (DAQ board) 20 acquires ultrasonic signals $S1_n$ in an ultrasonic frequency band (20 KHz to 200 KHz) by using ultrasonic signals sensed by the ultrasonic sensor array 10 as a first sampling frequency $f_{s1}$; a low-frequency conversion signal generating step (S130), in which a main board 30 heterodyne-converts the ultrasonic signals $S1_n$ acquired in step S20, and generates low-frequency conversion signals $S2_n$ in a sound wave band (20 Hz to 20 KHz) based on the ultrasonic signals $S1_n$; a second data acquiring step (S140), in which the main board 30 re-samples the low-frequency conversion signals $S2_n$ generated in step S30 as a second sampling frequency $f_{s2}$, which is smaller than the first sampling frequency $f_{s1}$ to acquire a low-frequency re-sampling signal $x_n$; and a sound field visualizing step (S200), in which the main operation board 30 beam-forms the low-frequency re-sampling signals $x_n$ and a display device 70 performs the sound field visualization, in which the ultrasonic sound source is visualized by converting an ultrasonic signal in a band of 20 KHz or more into a sound wave band signal without distorting sound source location information of the sound source of the radiation ultrasonic wave and then re-sampling and beam forming the converted ultrasonic signal.

## BRIEF DESCRIPTION OF THE DRAWINGS

FIGS. 1A and 1B are flowcharts of a radiation ultrasonic wave visualization method according to the present invention.
FIG. 2 is a configuration diagram of a radiation ultrasonic wave visualization apparatus according to the present invention.
FIG. 3 is a conceptual view of a radiation ultrasonic wave visualization sensor coordinate and a virtual plane coordinate according to the present invention.
FIG. 4 is a conceptual view of a radiation time delay summation according to the present invention.

## DETAILED DESCRIPTION

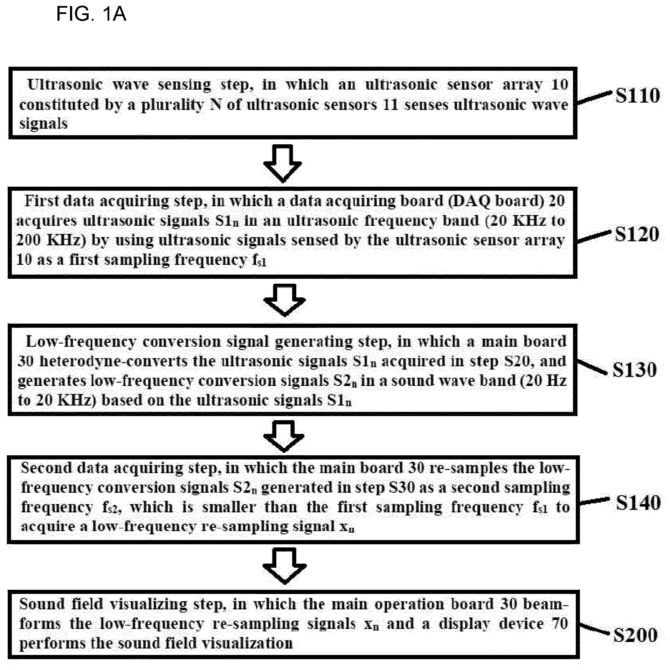

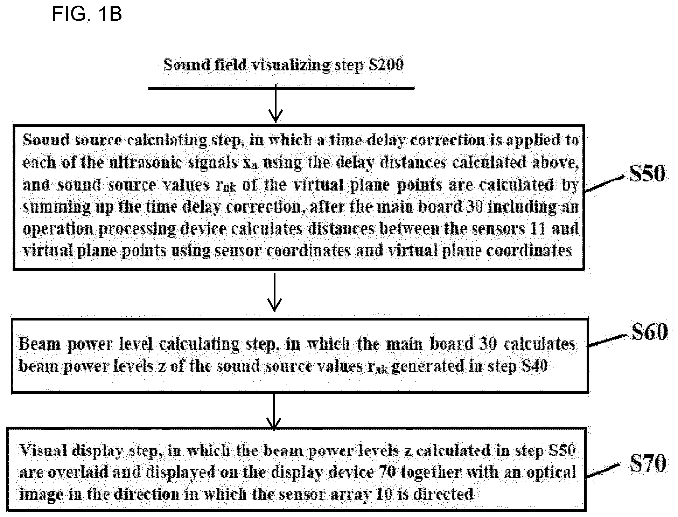

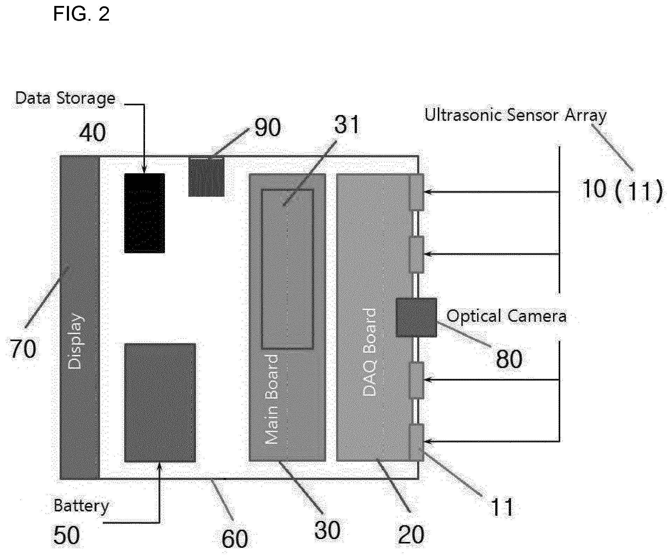

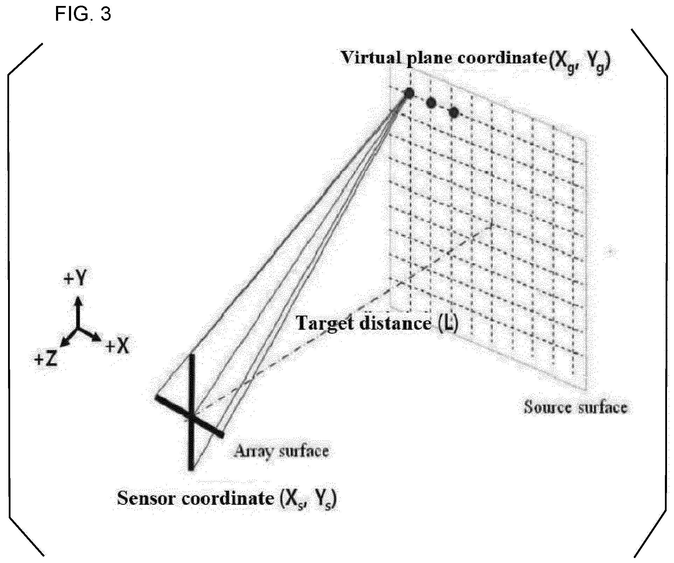

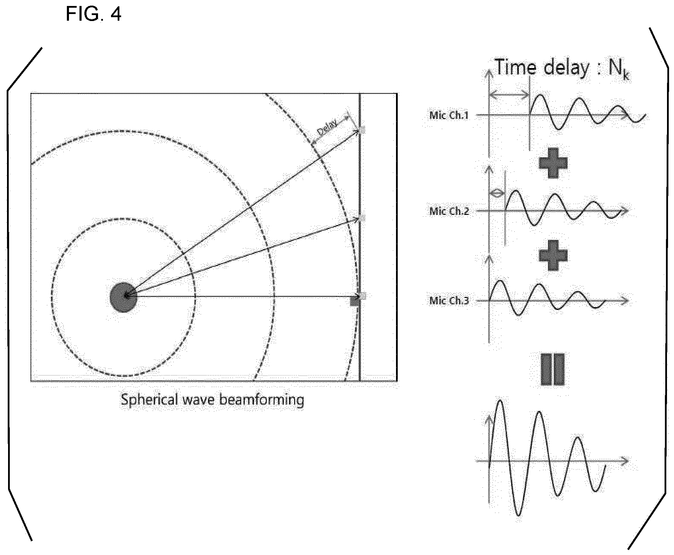

Hereinafter, a radiation ultrasonic wave visualization method and an electronic recording medium having a program for performing the radiation ultrasonic wave visualization method, which is recorded therein will be described in detail with reference to the accompanying drawings. FIG. 1 is a flowchart of a radiation ultrasonic wave visualization method according to the present invention, FIG. 2 is a configuration diagram of a radiation ultrasonic wave visualization apparatus according to the present invention, FIG. 3 is a conceptual view of a radiation ultrasonic wave visualization sensor coordinate and a virtual plane coordinate according to the present invention, and FIG. 4 is a conceptual view of a radiation time delay summation according to the present invention.

As illustrated in FIGS. 1 to 4, a radiation ultrasonic wave visualization method of the present invention as a method of visualizing an ultrasonic wave radiated by a sound source includes heterodyne-converting ultrasonic signals $S1_n$ in at least 20 KHz or more, which are acquired by an ultrasonic sensor array 10 constituted by a plurality of (N) ultrasonic sensors 11 and converts the ultrasonic signals $S1_n$ into a low-frequency signal $S2_n$ in a sound wave band (in detail, 20 Hz to 20 KHz) and thereafter, beamforming the low-frequency signal based on signals $x_n$ acquired by resampling the low-frequency signal beamformed or converted by using the converted low-frequency signals, thereby handling the low-frequency signal without distorting ultrasonic sound location information to reduce a data handling amount in a beamforming step.

As illustrated in FIGS. 1 to 4, the radiation ultrasonic wave visualization method of the present invention includes an ultrasonic wave sensing step (S110), a first data acquiring step (S120), a low-frequency conversion signal generating step (S130), a second data acquiring step (S140), and a sound filed visualizing step (S200).

First, in the ultrasonic wave sensing step (S110), an ultrasonic sensor array 10 constituted by a plurality N of ultrasonic sensors 11 senses ultrasonic signals. The ultrasonic sensor array 10 constituted by the plurality N of ultrasonic sensors 11 and orienting a radiation sound source senses the ultrasonic signals. The ultrasonic sensor array 10 constituted by the plurality N of ultrasonic sensors 11 and senses ultrasonic signals radiated from a facility while orienting the radiation sound source. The ultrasonic sensor array 10 may have a structure in which a plurality of MEMS microphones, ultrasonic transducers or ultrasonic sensors are mounted on a printed circuit board (PCB) on a planar surface or a flexible PCB on a curved surface. The ultrasonic sensor array 10 is exposed in front of the apparatus and arranged in a forward direction (one direction). Alternatively, the plurality of ultrasonic sensors 11 may be arranged at regular intervals on a sphere or a substantially ball-shaped polyhedron.

Next, in the first data acquiring step (S120), a data acquiring board (DAQ board) 20 acquires ultrasonic signals $S1_n$ in an ultrasonic frequency band (particularly, 20 KHz to 200 KHz) by using ultrasonic signals sensed by the ultrasonic sensor array 10 as a first sampling frequency $f_{s1}$.

Next, in the low-frequency conversion signal generating step (S130), a main board 30 heterodyne-converts the ultrasonic signals $S1_n$ acquired in step S20, and generates low-frequency conversion signals $S2_n$ in a sound wave band (20 Hz to 20 KHz) based on the ultrasonic signals $S1_n$.

Next, in the second data acquiring step (S140), the main board 30 re-samples the low-frequency conversion signals $S2_n$ generated in step S30 as a second sampling frequency $f_{s2}$, which is smaller than the first sampling frequency $f_{s1}$ to acquire a low-frequency re-sampling signal $x_n$.

A detailed equation for the signal $x_n$ is as follows.

$$x_n[s] = \sum_{s=0}^{S-1} x_n(t) \cdot \delta\left(t - \frac{s}{f_s}\right)$$

Herein, S: Sample Number, and $f_s$ : Sampling Rate(frequency).

A step of applying a band pass filter of predetermined ultrasonic frequency bands $f_1$ to $f_2$ (preset by the user) to the acquired ultrasonic signals $x_n$ may be further performed. In a filtering data $x_{nf}[s]$, $1 \le nf \le N$.

$$x_{nf}[s] = x_n[s] \cdot F[s]$$

Next, in the sound field visualizing step (S200), the main operation board 30 beam-forms the low-frequency re-sampling signals $x_n$ and a display device 70 performs the sound field visualization. The ultrasonic sound source is visualized by converting an ultrasonic signal in a band of 20 KHz or more into a sound wave band signal without distorting sound source location information of the sound source of the radiation ultrasonic wave and then re-sampling and beam forming the converted ultrasonic signal.

In the radiation ultrasonic wave visualization method according to the exemplary embodiment of the present invention, the first sampling frequency $f_{s1}$ is in a range of 20 KHz (40 KHz) to 200 KHz (400 KHz), and the second sampling frequency $f_{s2}$ is in a range of 20 Hz (40 Hz) to 20 KHz (40 KHz), and it is preferable that the first sampling frequency $f_{s1}$ is selected to be at least two times larger than the second sampling frequency $f_{s2}$ in terms of reduction of a data throughput.

In the range of the first sampling frequency $f_{s1}$ of 20 KHz (40 KHz) to 200 KHz (400 KHz), as the test result, it is possible to acquire ultrasonic sound source location information which is effective and required for the ultrasonic sensor detection performance and machinery failure currently released in this area, rotating machine breakdown, gas pipe gas leakage, and power equipment diagnosis monitoring. Further, as the test result, it can be seen that the data throughput may be appropriately reduced in the range of the sampling frequency $f_{s2}$ of 20 Hz (40 Hz) to 20 KHz. If the sampling frequency is too large, more data processing is needed, and if the sampling frequency is too small, the ultrasonic area sound source information is lost.

### <Sound field visualizing step (S200)>

As described above, in the sound field visualizing step (S200), the main operation board 30 beam-forms the low-frequency re-sampling signals $x_n$ and the display device 70 performs the sound field visualization, and it will be described in more detail.

The sound field visualizing step (S200) largely includes a sound source value calculation step (S50) by a time delay sum, a beam power level calculating step (S60), and a visual display step (S70).

First, in the sound source value calculating step (S50), the main board 30 including an operation processing device calculates distances between the sensors 11 and virtual plane points using sensor coordinates and virtual plane coordinates. Thereafter, time delay correction is applied to each of the ultrasonic signals $x_n$ using the delay distances calculated above, and sound source values $r_{nk}$ of the virtual plane points are calculated by summing up the time delay corrections.

FIG. 3 is a diagram illustrating a relationship between the sensor coordinate and the virtual plane coordinate. As illustrated in FIG. 3, a distance $d_k$ between the sensor coordinate (Xs, Ys) and the virtual plane coordinate (Xg, Yg) is calculated as follows. When the distance L is 1 m, the operation of $+L^2$ is represented by $+1$ operation.

$$d_k = \sqrt{(X_s - X_g)^2 + (Y_s - Y_g)^2 + L^2}$$

FIG. 4 is a conceptual diagram of a radiation ultrasonic wave visualization time delay summation of the present invention. Subsequently, in the sound source value calculating step (S50), first, a time delay correction is applied to each of the ultrasonic signals xn using the calculated delay distances, and sound source values $r_{nk}$ of M virtual plane points are calculated by summing up the time delay corrections.

First, a delay sample number is calculated. The time delay is calculated using a distance between the sensor and the virtual plane and a sound speed and the delay sample number is calculated by the calculated time delay. The details are as follows.

$$\tau_k = \frac{d_k}{c} \quad \text{(Time delay),}$$

$$N_k = f_s \cdot \tau_k = f_s \cdot \frac{d_k}{c} = \frac{f_s}{c} \cdot d_k = C_d \cdot d_k$$

$$C_d = \frac{f_s}{c}$$

Herein, $C_d$ represents a time delay coefficient and c is a sound speed. $N_k$ represents the delay sample number.

Next, the time delay is compensated by using the delay sample number and summed up. In this case, a correction coefficient for each sensor is applied.

$$\check{r}_{nk}[s] = \sum_{n=0}^{N-1} \alpha_n \cdot x_{nf}[s] \cdot \delta[s - N_k]$$

$\alpha_n$ : Weighting Factor

Herein, $1 \leq nk \leq M$. M is the number of all elements in rows and columns on a virtual plane coordinate.

Next, the beam power level calculating step (S60) for calculating the beam power levels z of the generated sound source values $r_{nk}$ is performed.

$$z_k = \frac{1}{N} \sum_{S=0}^{S-1} \check{r}_{nk}^2[s]$$

In the visual display step (S70), the beam power levels z calculated in step S50 are overplayed and displayed on the display device 70 together with an optical image in the direction which the sensor array 10 faces.

**<Apparatus>**

An apparatus that performs the method of the present invention will be described in detail. The apparatus for performing the method of the present invention includes an ultrasonic sensor array 10, a data acquisition board (DAQ board) 20, a main board 30, a data storage medium 40, a battery 50, a plastic body case 60, and a display device 70.

As illustrated in FIG. 2, the ultrasonic sensor array 10 is constituted by a plurality N of ultrasonic sensors 11 and senses ultrasonic signals radiated from a facility while orienting the radiation sound source. The ultrasonic sensor array 10 may have a structure in which a plurality of MEMS microphones, ultrasonic transducers or ultrasonic wave sensors are mounted on a printed circuit board (PCB) on a planar surface or a flexible PCB on a curved (three-dimensional) surface, a sphere, a substantially ball-shaped polyhedron, a hemisphere, and a rear-opened convex curved surface.

An electronic circuit for acquiring the ultrasonic signals $x_n$ using ultrasonic signals sensed from the ultrasonic sensor array 10 as a sampling frequency $f_s$ is mounted on the substrate of the DAQ board 20. The DAQ board 20 performs sampling and may include a signal amplification circuit.

In the main board 30, an operation processing device 31 that processes digital (alternatively, analog) ultrasonic signals received from the DAQ board 20 is mounted on the substrate and transmits the processed ultrasonic sound source information to the display device 70. The data storage medium 40 stores data processed in the operation processing device 31 of the main board 30.

The apparatus includes an optical camera 80 for picking up an image of a direction in which the ultrasonic sensor array 10 is directed and transmitting the image to the main board 30. The display device 70 visually displays the data processed by the operation processing unit 31 of the main board 30 and is integrally installed in the plastic body case 60. Alternatively, the display device 70 is integrally fixed to the plastic body case 60 so as to be exposed to the outside of the plastic body case 60.

The battery 50 supplies electric power to the data acquisition board 20, the main board 30 and the display device 70, and it is preferable that the battery 50 is installed in a detachable and rechargeable state inside the plastic body case 60. However, the battery may be a separate portable rechargeable battery which is located outside the plastic body case 60 and supplies electric power to the data acquisition board 20 and the main board 30 by electric wires. Alternatively, both an internal battery and an external auxiliary battery may be provided and used.

The plastic body case 60 is formed of a hard material for fixing the ultrasonic sensor array 10, the data acquisition board 20, the main board 30 and the data storage medium 40. The plastic body case 60 supports the array 10 constituted by the plurality of ultrasonic sensors 11 electrically connected to each other, or supports the ultrasonic sensor array 10 by supporting and fixing an ultrasonic sensor array PCB mounted on a flat or curved plate on which the ultrasonic sensors 11 are mounted. The inside of the plastic body case 60 has a hollow chamber, and the data acquisition board 20 and the main board 30 having an operation processing capability are fixedly installed in the hollow chamber.

The display device 70 visually displays the data processed by the operation processing unit 31 of the main board 30 and is integrally installed in the plastic body case 60. Alternatively, the display device 70 is integrally fixed to the plastic body case 60 so as to be exposed to the outside of the plastic body case 60.

The present invention includes an electronic recording medium on which a program is recorded for the radiation ultrasound visualization method, wherein the electronic recording medium is an electronic device including a CPU for executing a program, a hard disk on which a program is stored, a stationary memory, a removable memory and the like.

The present invention has been described in association with the above-mentioned preferred embedment, but the scope of the present invention is not limited to the embodiment and the scope of the present invention is determined by the appended claims, and thereafter, the scope of the present invention will includes various modifications and transformations included in an equivalent range to the present invention.

Reference numerals disclosed in the appended claims are just used to assist appreciation of the present invention and it is revealed that the reference numerals do not influence analysis of the claims and it should not be narrowly analyzed by the disclosed reference numerals.

**Claims**

1. A radiation ultrasonic wave visualization method in which an ultrasonic wave radiated by a sound source is visualized, comprising:

   heterodyne-converting ultrasonic signals $S1_n$ in a band of at least 20 KHz or more, which are acquired by an ultrasonic sensor array 10 constituted by a plurality of (N) ultrasonic sensors 11 and converting the ultrasonic signals $S1_n$ into a low-frequency signal $S2_n$ and thereafter,
   beamforming the converted low-frequency signals or beamforming the converted low-frequency signals based on resampling signals $x_n$,

thereby handling the low-frequency signals without distorting ultrasonic sound location information to reduce a data handling amount in the beamforming step.

2. A radiation ultrasonic wave visualization method, comprising:

an ultrasonic wave sensing step (S110), in which an ultrasonic sensor array 10 constituted by a plurality N of ultrasonic sensors 11 senses ultrasonic wave signals;

a first data acquiring step (S120), in which a data acquiring board (DAQ board) 20 acquires ultrasonic signals $S1_n$ in an ultrasonic frequency band (20 KHz to 200 KHz) by using ultrasonic signals sensed by the ultrasonic sensor array 10 as a first sampling frequency $f_{s1}$;

a low-frequency conversion signal generating step (S130), in which a main board 30 heterodyne-converts the ultrasonic signals $S1_n$ acquired in step S120, and generates low-frequency conversion signals $S2_n$ in a sound wave band (20 Hz to 20 KHz) based on the ultrasonic signals $S1_n$;

a second data acquiring step (S140), in which the main board 30 re-samples the low-frequency conversion signals $S2_n$ generated in step S130 as a second sampling frequency $f_{s2}$, which is smaller than the first sampling frequency $f_{s1}$ to acquire a low-frequency re-sampling signal $X_n$; and

a sound field visualizing step (S200), in which the main operation board 30 beam-forms the low-frequency re-sampling signals $x_n$ and a display device 70 performs the sound field visualization,

wherein the ultrasonic sound source is visualized by converting an ultrasonic signal in a band of 20 KHz or more into a sound wave band signal without distorting sound source location information of the sound source of the radiation ultrasonic wave and then re-sampling and beam forming the converted ultrasonic signal.

3. The radiation ultrasonic wave visualization method of claim 2, wherein the first sampling frequency $f_{s1}$ is in a range of 20 KHz to 200 KHz, the second sampling frequency $f_{s2}$ is in a range of 20 Hz to 20 KHz, and the first sampling frequency $f_{s1}$ is selected to be at least two times larger than the second sampling frequency $f_{s2}$.

4. The radiation ultrasonic wave visualization method of claim 2, wherein
the sound field visualizing step (S200) includes

a sound source calculating step (S50), in which a time delay correction is applied to each of the ultrasonic signals $x_n$ using the delay distances calculated above, and sound source values $r_{nk}$ of the virtual plane points are calculated by summing up the time delay correction after the main board 30 including an operation processing device calculates distances between the sensors 11 and virtual plane points using sensor coordinates and virtual plane coordinates;

a beam power level calculating step (S60), in which the main board 30 calculates beam power levels z of the sound source values $r_{nk}$ generated in step S40; and

a visual display step (S70), in which the beam power levels z calculated in step S50 are overplayed and displayed on the display device 70 together with an optical image in the direction in which the sensor array 10 is directed.

5. The radiation ultrasonic wave visualization method of claim 2, further comprising:

between the second data acquiring step (S140) and the sound field visualizing step (S200),
applying a band pass filter in predetermined frequency bands $f_1$ and $f_2$ to the ultrasonic signals $x_n$ acquired in step S140.

6. An electronic apparatus performing the radiation ultrasonic wave visualization method of any one of claims 1 to 5.

FIG. 1A

| | |
|---|---|
| Ultrasonic wave sensing step, in which an ultrasonic sensor array 10 constituted by a plurality N of ultrasonic sensors 11 senses ultrasonic wave signals | S110 |

⇩

| | |
|---|---|
| First data acquiring step, in which a data acquiring board (DAQ board) 20 acquires ultrasonic signals $S1_n$ in an ultrasonic frequency band (20 KHz to 200 KHz) by using ultrasonic signals sensed by the ultrasonic sensor array 10 as a first sampling frequency $f_{s1}$ | S120 |

⇩

| | |
|---|---|
| Low-frequency conversion signal generating step, in which a main board 30 heterodyne-converts the ultrasonic signals $S1_n$ acquired in step S20, and generates low-frequency conversion signals $S2_n$ in a sound wave band (20 Hz to 20 KHz) based on the ultrasonic signals $S1_n$ | S130 |

⇩

| | |
|---|---|
| Second data acquiring step, in which the main board 30 re-samples the low-frequency conversion signals $S2_n$ generated in step S30 as a second sampling frequency $f_{s2}$, which is smaller than the first sampling frequency $f_{s1}$ to acquire a low-frequency re-sampling signal $x_n$ | S140 |

⇩

| | |
|---|---|
| Sound field visualizing step, in which the main operation board 30 beam-forms the low-frequency re-sampling signals $x_n$ and a display device 70 performs the sound field visualization | S200 |

FIG. 1B

Sound field visualizing step S200

Sound source calculating step, in which a time delay correction is applied to each of the ultrasonic signals $x_n$ using the delay distances calculated above, and sound source values $r_{nk}$ of the virtual plane points are calculated by summing up the time delay correction, after the main board 30 including an operation processing device calculates distances between the sensors 11 and virtual plane points using sensor coordinates and virtual plane coordinates — S50

Beam power level calculating step, in which the main board 30 calculates beam power levels z of the sound source values $r_{nk}$ generated in step S40 — S60

Visual display step, in which the beam power levels z calculated in step S50 are overlaid and displayed on the display device 70 together with an optical image in the direction in which the sensor array 10 is directed — S70

FIG. 2

FIG. 3

Virtual plane coordinate $(X_g, Y_g)$

Target distance (L)

+Y

+Z +X

Source surface

Array surface

Sensor coordinate $(X_s, Y_s)$

FIG. 4

Spherical wave beamforming

Time delay : $N_k$

Mic Ch.1

Mic Ch.2

Mic Ch.3

## INTERNATIONAL SEARCH REPORT

| International application No. |
| --- |
| PCT/KR2018/005578 |

| A. | CLASSIFICATION OF SUBJECT MATTER |
| --- | --- |

*G01N 29/36(2006.01)i, G01N 29/06(2006.01)i, G01N 29/22(2006.01)i*

According to International Patent Classification (IPC) or to both national classification and IPC

| B. | FIELDS SEARCHED |
| --- | --- |

Minimum documentation searched (classification system followed by classification symbols)

G01N 29/36; G01N 33/00; G01P 15/00; G01S 15/89; G01J 5/48; G01R 31/12; G01R 31/02; G01H 11/06; H01F 27/00; A61B 8/00; G01N 29/06; G01N 29/22

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: sound source, ultrasonic sensor array, heterodyne conversion, low-frequency signal, beamforming

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y | KR 10-1406135 B1 (JEONG, Eui Jong) 12 June 2014<br>See abstract, paragraphs [0020], [0021], [0028], claims 1-3 and figures 1-4. | 1-6 |
| Y | KR 10-2016-0046777 A (ALPINION MEDICAL SYSTEMS CO., LTD.) 29 April 2016<br>See paragraphs [0027], [0028], claims 1, 2 and figures 1, 2. | 1-6 |
| Y | KR 10-1522996 B1 (CORESENSE. CO., LTD.) 27 May 2015<br>See abstract, paragraphs [0024]-[0028], [0053], [0058], claims 1, 7, 8 and figure 1. | 2-5 |
| Y | KR 10-2010-0128855 A (SM INSTRUMENTS CO., LTD.) 08 December 2010<br>See paragraphs [0040], [0041], claim 1 and figure 3. | 4 |
| A | KR 10-2010-0029338 A (HA, Joo Young et al.) 17 March 2010<br>See claim 1 and figure 2. | 1-6 |

| ☐ | Further documents are listed in the continuation of Box C. | ☒ | See patent family annex. |
| --- | --- | --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 11 SEPTEMBER 2018 (11.09.2018) | **11 SEPTEMBER 2018 (11.09.2018)** |

| Name and mailing address of the ISA/KR | Authorized officer |
| --- | --- |
| Korean Intellectual Property Office<br>Government Complex Daejeon Building 4, 189, Cheongsa-ro, Seo-gu,<br>Daejeon, 35208, Republic of Korea<br>Facsimile No. +82-42-481-8578 | Telephone No. |

Form PCT/ISA/210 (second sheet) (January 2015)

INTERNATIONAL SEARCH REPORT
Information on patent family members

| International application No. |
| --- |
| **PCT/KR2018/005578** |

| Patent document cited in search report | Publication date | Patent family member | Publication date |
| --- | --- | --- | --- |
| KR 10-1406135 B1 | 12/06/2014 | NONE | |
| KR 10-2016-0046777 A | 29/04/2016 | CN 105520752 A | 27/04/2016 |
| | | JP 2016-077907 A | 16/05/2016 |
| | | KR 10-2016-0046669 A | 29/04/2016 |
| | | US 2016-0109563 A1 | 21/04/2016 |
| KR 10-1522996 B1 | 27/05/2015 | NONE | |
| KR 10-2010-0128855 A | 08/12/2010 | KR 10-1059081 B1 | 24/08/2011 |
| KR 10-2010-0029338 A | 17/03/2010 | KR 10-1050870 B1 | 20/07/2011 |

Form PCT/ISA/210 (patent family annex) (January 2015)

**EP 3 627 148 A1**

**REFERENCES CITED IN THE DESCRIPTION**

**Patent documents cited in the description**

- KR 1020170060418
- KR 101477755